# EUROPEAN PATENT SPECIFICATION

(11) **EP 2 130 048 B1**
(45) Date of publication and mention of the grant of the patent: **18.04.2012**
(21) Application number: 08716640.1
(22) Date of filing: 19.03.2008
(51) Int. Cl.: G01N 33/574, G01N 33/573

(54) **APEX AS A MARKER FOR LUNG CANCER**
APEX ALS MARKER FÜR LUNGENKREBS
APEX COMME MARQUEUR POUR LE CANCER DU POUMON

(30) Priority: 23.03.2007 EP 07006079
(43) Date of publication of application: 09.12.2009
(73) Proprietor: F. Hoffmann-La Roche AG, 4070 Basel (CH); Roche Diagnostics GmbH, 68305 Mannheim (DE)
(72) Inventor: HAGMANN, Marie-Luise, 82377 Penzberg (DE); KARL, Johann, 82380 Peissenberg (DE); KLOECKNER, Julia, 81379 München (DE); ROESSLER, Markus, 82110 Germering (DE); TACKE, Michael, 80689 München (DE); THIEROLF, Michael, 88400 Biberach (DE)
(74) Representative: Weiss, Wolfgang
(86) International application number: PCT/EP2008/002224
(87) International publication number: WO 2008/116592

(56) References cited:
- WO-A-97/47971
- WO-A-02/076280
- PUGLISI F ET AL: "Prognostic significance of Ape1/ref-1 subcellular localization in non-small cell lung carcinomas." ANTICANCER RESEARCH, vol. 21, no. 6A, November 2001 (2001-11), pages 4041-4049, XP008083956 cited in the application
- SCHNEIDER J: "Tumor markers in detection of lung cancer." ADVANCES IN CLINICAL CHEMISTRY, vol. 42, 2006, pages 1-41, XP008083971
- MOLINA R ET AL: "Pro-gastrin-releasing peptide (proGRP) in patients with benign and malignant diseases: comparison with CEA, SCC, CYFRA 21-1 and NSE in patients with lung cancer." ANTICANCER RESEARCH, vol. 25, no. 3A, May 2005 (2005-05), pages 1773-1778, XP008083991

### SEQUENCE LISTING

<110> F. Hoffmann-La Roche AG
<120> APEX as a marker for lung cancer
<130> 39943P EP
<160> 4
<170> PatentIn version 3.3
<210> 1
   <211> 318
   <212> PRT
   <213> Homo sapiens
<220>
   <221> PEPTIDE
   <222> (1)..(318)
   <223> APEX
<400> 1
   Met Pro Lys Arg Gly Lys Lys Gly Ala Val Ala Glu Asp Gly Asp Glu
   1               5                   10                  15
   Leu Arg Thr Glu Pro Glu Ala Lys Lys Ser Lys Thr Ala Ala Lys Lys
               20                  25                  30
   Asn Asp Lys Glu Ala Ala Gly Glu Gly Pro Ala Leu Tyr Glu Asp Pro
           35                  40                  45
   Pro Asp Gln Lys Thr Ser Pro Ser Gly Lys Pro Ala Thr Leu Lys Ile
       50                  55                  60
   Cys Ser Trp Asn Val Asp Gly Leu Arg Ala Trp Ile Lys Lys Lys Gly
   65                  70                  75                  80
   Leu Asp Trp Val Lys Glu Glu Ala Pro Asp Ile Leu Cys Leu Gln Glu
                   85                  90                  95
   Thr Lys Cys Ser Glu Asn Lys Leu Pro Ala Glu Leu Gln Glu Leu Pro
               100                 105                 110
   Gly Leu Ser His Gln Tyr Trp Ser Ala Pro Ser Asp Lys Glu Gly Tyr
           115                 120                 125
   Ser Gly Val Gly Leu Leu Ser Arg Gln Cys Pro Leu Lys Val Ser Tyr
       130                 135                 140
   Gly Ile Gly Asp Glu Glu His Asp Gln Glu Gly Arg Val Ile Val Ala
   145                 150                 155                 160
   Glu Phe Asp Ser Phe Val Leu Val Thr Ala Tyr Val Pro Asn Ala Gly
                   165                     170                     175
   Arg Gly Leu Val Arg Leu Glu Tyr Arg Gln Arg Trp Asp Glu Ala Phe
               180                     185                     190
   Arg Lys Phe Leu Lys Gly Leu Ala Ser Arg Lys Pro Leu Val Leu Cys
           195                     200                     205
   Gly Asp Leu Asn Val Ala His Glu Glu Ile Asp Leu Arg Asn Pro Lys
       210                     215                     220
   Gly Asn Lys Lys Asn Ala Gly Phe Thr Pro Gln Glu Arg Gln Gly Phe
   225                     230                     235                     240
   Gly Glu Leu Leu Gln Ala Val Pro Leu Ala Asp Ser Phe Arg His Leu
                   245                     250                     255
   Tyr Pro Asn Thr Pro Tyr Ala Tyr Thr Phe Trp Thr Tyr Met Met Asn
               260                     265                     270
   Ala Arg Ser Lys Asn Val Gly Trp Arg Leu Asp Tyr Phe Leu Leu Ser
           275                     280                     285
   His Ser Leu Leu Pro Ala Leu Cys Asp Ser Lys Ile Arg Ser Lys Ala
       290                     295                     300
   Leu Gly Ser Asp His Cys Pro Ile Thr Leu Tyr Leu Ala Leu
   305                     310                     315
<210> 2
   <211> 96
   <212> DNA
   <213> Artificial
<220>
   <223> forward primer LC38for-EcoRI
<220>
   <221> misc_feature
   <222> (1)..(96)
<400> 2
   acgtacgtga attcattaaa gaggagaaat taactatgag aggatcgcat caccatcacc      60
   atcacattga aggccgtccg aagcgtggga aaaagg                                96
<210> 3
   <211> 43
   <212> DNA
   <213> Artificial
<220>
   <223> reverse primer LC38rev-BamHI
   <220>
   <221> misc_feature
   <222> (1)..(43)
<400> 3
   cgtacgtgga tcctcattac agtgctaggt atagggtgat agg 43
<210> 4
   <211> 14
   <212> PRT
   <213> Artificial
<220>
   <223> peptide extension
<220>
   <221> PEPTIDE
   <222> (1)..(14)
<400> 4
   Met Arg Gly Ser His His His His His His Ile Glu Gly Arg
   1               5                   10

## Description

The present invention relates to a method aiding in the assessment of pulmonary or lung cancer (= LC) and in particular in the assessment of non-small cell lung carcinoma (NSCLC). It discloses the use of the AP endonuclease (= APEX) as a marker of LC, particularly of NSCLC. Furthermore, it especially relates to a method for assessing lung cancer from a liquid sample, derived from an individual by measuring APEX in said sample. Measurement of APEX can, e.g., be used in the early detection of lung cancer or in the surveillance of patients who undergo surgery.

Cancer remains a major public health challenge despite progress in detection and therapy. Amongst the various types of cancer, LC is a frequent cancer in the Western world and among the most frequent causes of cancer-related mortality. This is in large part due to the diagnostic gap for early detection of the disease. LC is largely asymptomatic in its early stages. The majority of all lung cancers is detected at a late stage when the disease has already become inoperable.

The majority of LC tumors can be divided into small cell lung carcinoma (SCLC) and non-small cell lung carcinoma (NSCLC). SCLC accounts for about 20-25% of all lung cancer cases. SCLC is an aggressive neuroendocrine type of LC and has a very poor prognosis even if detected in early stages. SCLC is rarely amenable to curative treatment by resection. Because of the speed with which the disease progresses, SCLC is generally categorized using only two stages, i.e. limited and extensive disease, rather than the more complex TNM staging system (see below). About 75-80% of LC cases are grouped into the class of NSCLC including squamous cell carcinoma (carcinoma = CA), adeno CA (comprising the subclasses of acinar CA, papillary CA, bronchoalveolar tumor, solid tumor, and mixed subtypes), and large cell carcinoma (comprising the subclasses of giant cell tumors, clear cell CA, adenosquamous CA, and undifferentiated CA). NSCLC, if detected at late stages, also has a very poor prognosis. The staging of cancer is the classification of the disease in terms of extent, progression, cell type and tumor grade. It groups cancer patients so that generalizations can be made about prognosis and the choice of therapy.

Today, the TNM system is the most widely used classification system based on the anatomical extent of cancer. It represents an internationally accepted, uniform staging system. There are three basic variables: T (the extent of the primary tumor), N (the status of regional lymph nodes) and M (the presence or absence of distant metastases). The TNM criteria are published by the UICC (International Union Against Cancer), edition, 1997 (Sobin, L.H., and Fleming, I.D., TNM 80 (1997) 1803-4).

Surgical resection of the primary tumor is widely accepted as the treatment of choice for early stage NSCLC. With the progression of NSCLC and, more specifically, the transition from stage IIIa (T3N1M0, T1N2M0, T2N2M0, T3N2M0) to IIIb (T4N0M0, T4N1M0, T4N2M0), a significant shift in the physician's approach is precipitated. However, if the cancer is detected during the more early stages (Ia - IIIa; preferably up to stage T3N1M0), the five-year survival rate varies between 35% and 80%. Detection at stage la ((T1N0M0); small tumor size, no metastasis) has evidently the best prognosis with a five-year survival of up to 80%.

Surgery is rarely, if ever, used in the management of stage IIIb-IV of NSCLC. Stage IV corresponds to distant metastasis, i.e. spread of the disease beyond the lungs and regional lymph nodes. The five-year survival rate in the later stages III and IV drops to between less than 15% and 1%, respectively.

What is especially important is, that early diagnosis of NSCLC translates to a much better prognosis. Patients diagnosed as early as in stage Ia (T1 N0M0), Ib (T2N0M0), IIa (T1N1M0), IIb, (T3N0M0), and IIIa (T3N1M0), if treated properly have an up to 80% chance of survival 5 years after diagnosis. This has to be compared to a 5-years survival rate of less than 1 % for patients diagnosed once distant metastases are already present.

In the sense of the present invention early assessment of LC refers to an assessment at a tumor stage of between Ia and IIIa, as defined above.

It is preferred, that LC is assessed at a stage of between Ia and IIIa.

Most lung cancers are detected when they become symptomatic. Current detection methods include chest x-ray, spiral computer tomography, sputum cytology and bronchioscopy. However, there is controversy regarding the suitability of these means for mass screenings.

A number of serum tumor markers for lung cancers are in clinical use. The soluble 30 kDa fragment of cytokeratin 19 (CYFRA 21-1), carcinoembryonic antigen (CEA), neuron-specific enolase (NSE), and squamous cell carcinoma antigen (SCC) are the most prominent LC markers. However, none of them meets the criteria for sensitivity and specificity required for a screening tool (Thomas, L., Labor und Diagnose (2000) TH Books Verlagsgesellschaft, Frankfurt/Main, Germany).

In order to be of clinical utility, a new diagnostic marker as a single marker should be comparable to other markers known in the art, or better. Or, a new marker should lead to a progress in diagnostic sensitivity and/or specificity either if used alone or in combination with one or more other markers, respectively. The diagnostic sensitivity and/or specificity of a test is best assessed by its receiver-operating characteristics, which will be described in detail below.

Whole blood, serum or plasma are the most widely used sources of sample in clinical routine. The identification of an early LC tumor marker that would aid in the reliable cancer detection or provide early prognostic information could lead to a method that would greatly aid in the diagnosis and in the management of this disease. Therefore, an urgent clinical need exists to improve the in vitro assessment of LC. It is especially important to improve the early diagnosis of LC, since for patients diagnosed early on chances of survival are much higher as compared to those diagnosed at a progressed stage of disease.

The clinical utility of biochemical markers in lung cancer has recently been reviewed (Duffy, M.J., Critical Reviews in Clinical Laboratory Sciences 38 (2001) 225-262).

CYFRA 21-1 is currently regarded to be the best of the presently known tumor markers for lung cancer. Even though not organ-specific it is predominantly found in lung tissue. Sensitivity of CYFRA 21-1 for lung cancer is described to be between 46-61% at a specificity of 95% towards other benign lung diseases. Increased serum levels of CYFRA 21-1 are also associated with pronounced benign liver diseases, renal insufficiency and invasive bladder cancer. CYFRA 21-1 testing is recommended for postoperative therapy surveillance.

CEA belongs to the group of carcinofetal antigens, usually produced during embryogenesis. CEA is not organ-specific and predominantly used for monitoring of colorectal cancer. Besides malignancies, also several benign diseases such as cirrhosis, bronchitis, pancreatitis and autoimmune diseases are associated with increased CEA serum levels. At 95% specificity towards benign lung diseases its sensitivity for lung cancer is reported to be 29-44%. A preferred use of CEA is therapy surveillance of lung cancer.

NSE is a tumor marker for SCLC. Generally, increased NSE serum levels are found in association with neuroectodermal and neuroendocrine tumors. Increased serum levels are also found in patients with benign lung diseases and cerebral diseases, such as meningitis or other inflammatory diseases of the brain, and traumatic injuries to the head. While the sensitivity for SCLC at 95% specificity is reported to be 60-87%, the performance of NSE testing for NSCLC is poor (sensitivity of 7-25%). NSE is recommended for therapy surveillance of SCLC.

ProGRP is a tumor marker, useful in the detection and monitoring of SCLC. Increased serum levels are also found in patients with nonmalignant lung/pleural diseases, such as idiopathic pulmonary fibrosis or sarcoidosis. While sensitivity for proGRP in the field of SCLC (at 95% specificity) is reported to be 47-86%, the performance of proGRP testing in the field of NSCLC is poor because the sensitivity is reported as being below 10%.

SCC was originally identified in squamous cell CA of the cervix. The sensitivity of SCC for LC in general is low (18-27%). Therefore, SCC testing is regarded to be not suitable for screening. However, due to a higher sensitivity for squamous cell CA, a preferred use for SCC is therapy surveillance, even though CYFRA 21-1 generally performs better.

With respect to marker profiles and aiming at improved diagnosis of lung cancer, a method was published (Schneider, J. et al. Int. J. Clin. Oncol. 7 (2002) 145-151) using fuzzy logic based classification algorithms to combine serum levels of CYFRA 21-1, NSE and C-reactive protein (CRP) which is a general inflammation marker. The authors report a sensitivity of 92% at a specificity of 95%. However in this study, for example the sensitivity of CYFRA 21-1 as a single tumor marker is reported to be at 72% at a specificity of 95%, which is significantly higher than in many other reported studies. Duffy, M.J., in Critical Reviews in Clinical Laboratory Sciences 38 (2001) 225-262 report a sensitivity of between 46% and 61%. This unusual high performance achieved by Schneider et al., raises some doubts and might be due to several facts. Firstly, the collective of control patients seems to be younger than the patients collective, i. e. the groups are not well age-matched, and the patient collective comprises many late stages. Secondly and even more critical, the performance of the algorithm is checked on the samples of the training set which were used for the determination of the fuzzy logic qualifiers. Hence, these qualifiers are strictly speaking "tailor-made" for this set and not applied to an independent validation set. Under normal circumstances, it has to be expected that the same algorithm applied to a larger, independent, and well balanced validation set will lead to a significantly reduced overall performance.

It was the task of the present invention to investigate whether a biochemical marker can be identified which may be used in assessing LC.

Surprisingly, it has been found that use of the marker APEX can at least partially overcome some of the problems of the markers presently known in the state of the art.

The present invention relates to a method for assessing lung cancer in vitro comprising measuring in a sample the presence and/or concentration of APEX in a sample selected from tissue extracts and body fluids, and using the measurement result, particularly the concentration determined in the assessment of lung cancer, wherein detection of increased presence and/or concentration of APEX is indicative for lung cancer.

The present invention is also directed to a method for assessing LC in vitro by biochemical markers, comprising measuring in a sample the presence and/or concentration of APEX in a sample selected from tissue extracts and body fluids and of one or more other marker of LC and using the measurement results, particularly concentrations determined in the assessment of LC, wherein detection of increased presence and/or concentration of APEX is indicative for lung cancer. It is preferred that the one or more other marker of LC is selected from the group consisting of CYFRA 21-1, CEA, NSE, proGRP and SCC.

The present invention, in a preferred embodiment, also relates to the use of a marker panel comprising at least APEX and CYFRA 21-1 in the in vitro assessment of LC in a sample selected from tissue extracts and body fluids, wherein detection of increased presence and/or concentration of APEX is indicative for lung cancer.

The present invention also relates to the use of a marker panel comprising at least APEX and CEA in the in vitro assessment of LC in a sample selected from tissue extracts and body fluids, wherein detection of increased presence and/or concentration of APEX is indicative for lung cancer.

The present invention also relates to the use of a marker panel comprising at least APEX and SCC in the in vitro assessment of LC in a sample selected from tissue extracts and body fluids wherein detection of increased presence and/or concentration of APEX is indicative for lung cancer.

The present disclosure also provides a kit for performing the method according to the present invention comprising at least the reagents required to specifically measure APEX and CYFRA 21-1, respectively, and optionally auxiliary reagents for performing the measurement.

The present disclosure also provides a kit for performing the method according to the present invention comprising at least the reagents required to specifically measure APEX and CEA, respectively, and optionally auxiliary reagents for performing the measurement.

The present disclosure also provides a kit for performing the method according to the present invention comprising at least the reagents required to specifically measure APEX and SCC, respectively, and optionally auxiliary reagents for performing the measurement.

In a preferred embodiment the present invention relates to a method for assessing lung cancer in vitro comprising measuring in a sample the presence and/or concentration of a) APEX in a sample selected from tissue extracts and body fluids , and b) optionally one or more other marker of lung cancer, and c) using the measurement results, particularly the concentrations determined in step (a) and optionally step (b) in the assessment of lung cancer wherein detection of increased presence and/or concentration of APEX is indicative for lung cancer..

The term "measurement" comprises a qualitative or a quantitative measurement of APEX in a sample. In a preferred embodiment the measurement is a qualitative or semi-quantitative measurement, i.e. it is determined whether APEX is present or absent or it is determined whether the concentration of APEX is above or below a cut-off value. As the skilled artisan will appreciate, in a Yes- (presence) or No- (absence) assay, the assay sensitivity is usually set to match the cut-off value. A cut-off value can for example be determined from the testing of a group of healthy individuals. Preferably the cut-off is set to result in a specificity of 90%, also preferred the cut-off is set to result in a specificity of 95%, or also preferred the cut-off is set to result in a specificity of 98%. Presence or a value above the cut-off value can for example be indicative for the presence of lung cancer. In a further preferred embodiment the measurement is a quantitative measurement. In this embodiment the concentration of APEX is correlated to an underlying diagnostic question like e.g. stage of disease, disease progression, or response to therapy.

The AP endonuclease APEX (Swiss-Prot. P27695) is characterized by the sequence given in SEQ ID NO:1. The unprocessed precursor molecule consists of 318 amino acids and has a molecular weight of 35.6 kDa. APEX is involved in DNA repair and excises the apurinic or apyrimidinic site of DNA strands. Such abasic sites are relative frequently generated either spontaneously or through chemical agents or by DNA glycosylases that remove specific abnormal bases.

AP sites are pre-mutagenic lesions that can prevent normal DNA replication so the cell contains systems to identify and repair such sites. (Gil Barzilay, lan D. Hickson, 1995, Bioessays 17 (18) pp 713-719*).* The 3 D structure was elucidated and the amino acids involved in endonuclease activity were identified *(*Barizilay G. et al., 1995, Nature structural biology 2 (7), pp 561-567*;* Gorman M.A. et al., 1997, EMBO Journal, 16 (21) pp 6548-58*;* Beernink P. et al., 2001, J. Mol. Biol. 307, pp 1023-1034*).* APEX is also a redox regulator of various transcription factors such as c-Fos, c-Jun, NF-KB and HIF-1. This activity seems to be independent from the endonuclease activity. Both functions are located on different domains of the protein *(*Gil Barzilay, lan D. Hickson, 1995, Bioessays 17 (18) pp 713-719*).* Phosphorylation of APEX by protein kinase C increases redox activity whereas the unphosphorylated form is involved in DNA-repair *(*Yacoub A. et al. (1997; Cancer Res. 57, pp 5457-59*).* One phosphorylation site, Y 261, (according to the Swissprot sequence) was identified by Rush J. et al., (2005, Nature Biotech, 23 (1) pp 94-101*).*

It was also observed that APEX activates p53 DNA-binding activity *(*Jayaraman L. et al., 1997, Genes Dev., 11 (5, p558-70*).* In vivo regulation of p53 by APEX was studied by Gaiddon et al., (1999, EMBO Journal, 18 (20), pp 5606-5621*).* The role of p53 in tumorigenesis is well established.

WO 97/47971 discloses the use of apurinic/apyrimidinic endonucleases as a marker for identifying a premalignant or malignant condition. The examples describe APEX staining in cervical cancer and prostate cancer tissue. A determination of APEX in tissue extracts or in body fluids is not described. Further, the document does not contain any data that APEX might be a marker associated with lung cancer.

WO 02/076280 discloses a method of determining a risk of a subject to develop cancer, wherein the level of a parameter indicative of a level of activity of a DNA repair/damage-preventing enzyme in a tissue of the subject is determined and, according to said level, the risk of the subject to develop the cancer is determined. The DNA damage-preventing enzyme may be APEX. The determination involves an OGG activity DNA repair test, wherein the DNA repair activity is tested using a synthetic oligonucleotide substrate. The sample is a protein extract prepared from human peripheral blood lymphocytes. Since DNA repair is a complex process, OGG activity cannot be strictly correlated with APEX. It was found that low OGG activity is a risk factor in lung cancer. A direct association of APEX with lung cancer is, however, not described.

WO 2006/091734 describes using peptide microarrays in the detection of an autoantibody profile and using such autoantibody profile in order to derive diagnostic or prognostic information. In total, 1480 epitopes are listed including 4 peptide sequences from APEX. The document does not describe any association of APEX with lung cancer.

Duguid et al., (Cancer Res. 55 (1995), 6097-6102) describe a determination of differential cellular and subcellular expression of human APEX by immunostaining, cytoplasmic and nuclear staining of APEX in several tissues, e.g. in the brain in the liver.

Tanner et al., (Gynecologic Oncol. 92 (2004), 568-577) describe an increase of nuclear APEX expression with the progression of ovarian carcinoma.

Kakolyris et al., (J. Pathol. 189 (1999), 351-357) describe that a nuclear localization of human APEX associates with prognosis in early operable NSCLC. In normal lung, staining for APEX was found to be both nuclear and cytoplasmic in the pneumocytes of the alveoli. Superficial ciliated cells of the bronchial epithelium showed cytoplasmic staining, while staining for the basal cells was mostly nuclear. Bronchial glandular cells demonstrated mixed nuclear and cytoplasmic staining. Lung carcinomas showed all patterns of expression for APEX. In squamous carcinomas, a significant indirect correlation was observed i.e. increased (positive) nuclear staining for APEX was paralleled by a decreased (negative) staining for p53. The authors conclude that nuclear APEX localisation may be relevant to its role as a DNA repair protein and/or as an activator of wild-type p53 and thus to the better outcome seen in a subgroup of patients.

Puglisi et al., (Anticancer Res. 21 (2001), 4041-4050 describe a potential role of subcellular APEX localization as a prognostic indicator in patients with NSCLC. In particular, cytoplasmic localization of the protein seems to be associated with a pure prognosis in patient subgroups.

Interestingly, none of the above documents suggests that a determination of APEX in tissue extracts and in body fluids would allow assessment of lung cancer. According to the prior art, only a subcellular staining of APEX would allow cancer assessment. Surprisingly, it was found in the present invention that a determination of the presence and/or amount of APEX in a tissue lysate sample and/or body fluid without subcellular analysis, particularly without determining subcellular localization, allows the assessment of lung cancer. Even more surprisingly it was found that an increased presence and/or concentration of APEX is associated with lung cancer.

As used herein, each of the following terms has the meaning associated with it in this section.

The articles "a" and "an" are used herein to refer to one or to more than one (i.e. to at least one) of the grammatical object of the article. By way of example, "a marker" means one marker or more than one marker. The term "at least" is used to indicate that optionally one or more further objects may be present. By way of example, a marker panel comprising at least (the markers) APEX and CYFRA 21-1 may optionally comprise one or more other marker.

The expression "one or more" denotes 1 to 50, preferably 1 to 20 also preferred 2, 3, 4, 5, 6, 7, 8, 9, 10, 12, or 15.

The term "marker" or "biochemical marker" as used herein refers to a molecule to be used as a target for analyzing a patient's test sample. In one embodiment examples of such molecular targets are proteins or polypeptides. Proteins or polypeptides used as a marker in the present invention are contemplated to include naturally occurring variants of said protein as well as fragments of said protein or said variant, in particular, immunologically detectable fragments. Immunologically detectable fragments preferably comprise at least 6, 7, 8, 10, 12, 15 or 20 contiguous amino acids of said marker polypeptide. One of skill in the art would recognize that proteins which are released by cells or present in the extracellular matrix may be damaged, e.g., during inflammation, and could become degraded or cleaved into such fragments. Certain markers are synthesized in an inactive form, which may be subsequently activated by proteolysis. As the skilled artisan will appreciate, proteins or fragments thereof may also be present as part of a complex. Such complex also may be used as a marker in the sense of the present invention. Variants of a marker polypeptide are encoded by the same gene, but may differ in their isoelectric point (=PI) or molecular weight (=MW), or both e.g., as a result of alternative mRNA or pre-mRNA processing. The amino acid sequence of a variant is to 95% or more identical to the corresponding marker sequence. In addition, or in the alternative a marker polypeptide or a variant thereof may carry a post-translational modification. Preferred posttranslational modifications are glycosylation, acylation, and/or phosphorylation.

Preferably the marker APEX is specifically measured from a sample by use of a specific binding agent.

A specific binding agent is, e.g., a receptor for APEX, a lectin binding to APEX or an antibody to APEX. A specific binding agent has at least an affinity of 10⁷ l/mol for its corresponding target molecule. The specific binding agent preferably has an affinity of 10⁸ l/mol or also preferred of 10⁹ l/mol for its target molecule. As the skilled artisan will appreciate the term specific is used to indicate that other biomolecules present in the sample do not significantly bind to the binding agent specific for APEX. Preferably, the level of binding to a biomolecule other than the target molecule results in a binding affinity which is at most only 10% or less, only 5% or less only 2% or less or only 1 % or less of the affinity to the target molecule, respectively. A preferred specific binding agent will fulfill both the above minimum criteria for affinity as well as for specificity.

A specific binding agent preferably is an antibody reactive with APEX. The term antibody refers to a polyclonal antibody, a monoclonal antibody, antigen binding fragments of such antibodies, single chain antibodies as well as to genetic constructs comprising the binding domain of an antibody.

Any antibody fragment retaining the above criteria of a specific binding agent can be used. Antibodies are generated by state of the art procedures, e.g., as described in Tijssen (Tijssen, P., Practice and theory of enzyme immunoassays, 11, Elsevier Science Publishers B.V., Amsterdam, the whole book, especially pages 43-78). In addition, the skilled artisan is well aware of methods based on immunosorbents that can be used for the specific isolation of antibodies. By these means the quality of polyclonal antibodies and hence their performance in immunoassays can be enhanced. (Tijssen, *P., supra,* pages 108-115).

For the achievements as disclosed in the present invention polyclonal antibodies raised in rabbits may be used. However, clearly also polyclonal antibodies from different species, e.g., rats or guinea pigs, as well as monoclonal antibodies can also be used. Since monoclonal antibodies can be produced in any amount required with constant properties, they represent ideal tools in development of an assay for clinical routine. The generation and the use of monoclonal antibodies to APEX in a method according to the present invention, respectively, represent yet other preferred embodiments.

As the skilled artisan will appreciate now, that APEX has been identified as a marker which is useful in the assessment of lung cancer, various immunodiagnostic procedures may be used to reach a result comparable to the achievements of the present invention. For example, alternative strategies to generate antibodies may be used. Such strategies comprise amongst others the use of synthetic peptides, representing an epitope of APEX for immunization. Alternatively, DNA immunization also known as DNA vaccination may be used.

For measurement the sample obtained from an individual is incubated with the specific binding agent for APEX under conditions appropriate for formation of a binding agent APEX-complex. Such conditions need not be specified, since the skilled artisan without any inventive effort can easily identify such appropriate incubation conditions. The amount of binding agent APEX-complex is measured and used in the assessment of lung cancer. As the skilled artisan will appreciate there are numerous methods to measure the amount of the specific binding agent APEX-complex all described in detail in relevant textbooks (cf., e.g., Tijssen P., *supra,* or Diamandis, E.P. and Christopoulos, T.K. (eds.), Immunoassay, Academic Press, Boston (1996)).

Preferably APEX is detected in a sandwich type assay format. In such assay a first specific binding agent is used to capture APEX on the one side and a second specific binding agent, which is labeled to be directly or indirectly detectable, is used on the other side.

A "marker of lung cancer" in the sense of the present invention is any marker that if combined with the marker APEX adds relevant information in the assessment of LC. The information is considered relevant or of additive value if at a given specificity the sensitivity, or if at a given sensitivity the specificity, respectively, for the assessment of LC can be improved by including said marker into a marker combination already comprising the marker APEX. Preferably the improvement in sensitivity or specificity, respectively, is statistically significant at a level of significance of p = .05, .02, .01 or lower. Preferably, the one or more other marker of LC is selected from the group consisting of CYFRA 21-1, CEA, NSE, proGRP and SCC.

The term "sample" as used herein refers to a biological sample obtained for the purpose of evaluation in vitro. In the methods of the present invention, the sample or patient sample comprise any body fluid or a tissue extract. Preferred test samples include blood, serum, plasma, sputum and bronchial lavage. Preferred samples are whole blood, serum, plasma, bronchial lavage or sputum, with plasma or serum being most preferred.

The term "assessing lung cancer" is used to indicate that the method according to the present invention will (alone or together with other markers or variables, e.g., the criteria set forth by the UICC (see above)) e.g., aid the physician to establish or confirm the absence or presence of LC or aid the physician in the prognosis, the detection of recurrence (follow-up of patients after surgery) and/or the monitoring of treatment, especially of chemotherapy.

As the skilled artisan will appreciate, any such assessment is made in vitro. The patient sample is discarded afterwards. The patient sample is solely used for the in vitro diagnostic method of the invention and the material of the patient sample is not transferred back into the patient's body. Typically, the sample is a liquid sample, e.g., whole blood, serum, or plasma.

In a preferred embodiment the present invention relates to a method for assessing LC in vitro by biochemical markers, comprising measuring in a sample the concentration of APEX in a sample selected from tissue extracts and body fluids and using the concentration determined in the assessment of LC wherein detection of increased presence and/or concentration of APEX is indicative for lung cancer..

The inventors of the present invention have surprisingly been able to detect the marker APEX in a significant percentage of samples derived from patients with LC. Even more surprising they have been able to demonstrate that the presence and/or concentration of APEX in such sample obtained from an individual can be used in the assessment of lung cancer.

The ideal scenario for diagnosis would be a situation wherein a single event or process would cause the respective disease as, e.g., in infectious diseases. In all other cases correct diagnosis can be very difficult, especially when the etiology of the disease is not fully understood as is the case for LC. As the skilled artisan will appreciate, no biochemical marker is diagnostic with 100% specificity and at the same time 100% sensitivity for a given multifactorial disease, for example for LC. Rather, biochemical markers, e.g., CYFRA 21-1, CEA, NSE, proGRP, SCC, or as shown here APEX can be used to assess with a certain likelihood or predictive value e.g., the presence, absence, or the severity of a disease. Therefore in routine clinical diagnosis, generally various clinical symptoms and biological markers are considered together in the diagnosis, treatment and management of the underlying disease.

Biochemical markers can either be determined individually or in a preferred embodiment of the invention they can be measured simultaneously using a chip or a bead based array technology. The concentrations of the biomarkers are then either interpreted independently, e.g., using an individual cut-off for each marker, or they are combined for interpretation.

In a further preferred embodiment the assessment of LC according to the present invention is performed in a method comprising measuring in a sample the presence and/or concentration of a) APEX in a sample selected from tissue extracts and body fluids , and b) one or more other marker of lung cancer, and c) using the measurement result, e.g. the concentrations determined in step (a) and step (b), respectively, in the assessment of lung cancer wherein detection of increased presence and/or concentration of APEX is indicative for lung cancer..

In the assessment of LC the marker APEX will be of advantage in one or more of the following aspects: screening; diagnostic aid; prognosis; monitoring of therapy such as chemotherapy, radiotherapy, and immunotherapy.

### Screening:

Screening is defined as the systematic application of a test to identify individuals e.g. at risk individuals, for indicators of a disease, e.g., the presence of lung cancer. Preferably the screening population is composed of individuals known to be at higher than average risk of lung cancer, like smokers, ex-smokers, and uranium-, quartz- or asbestos-exposed workers. In one preferred embodiment sputum is used as a sample in the screening for lung cancer.

For many diseases, no single biochemical marker in the circulation will ever meet the sensitivity and specificity criteria required for screening purposes. This appears to be also true for lung cancer. It has to be expected that a marker panel comprising a plurality of markers will have to be used in LC screening. The data established in the present invention indicate that the marker APEX will form an integral part of a marker panel appropriate for screening purposes. The present invention therefore relates to the use of APEX as one marker of a LC marker panel, i.e. a marker panel comprising APEX and one or more additional marker for LC screening purposes. The present data further indicate that certain combinations of markers will be advantageous in the screening for LC. Therefore the present invention also relates to the use of a marker panel comprising APEX and CYFRA 21-1, or of a marker panel comprising APEX and CEA, or of a marker panel comprising APEX and NSE, or of a marker panel comprising APEX and SCC, or of a marker panel comprising APEX and proGRP, or of a marker panel comprising APEX and two or more markers selected from the group consisting of CYFRA 21-1, CEA, NSE, proGRP and SCC, for the purpose of screening for LC.

### Diagnostic aid:

Markers may either aid the differential diagnosis of benign vs. malignant disease in a particular organ, help to distinguish between different histological types of a tumor, or to establish baseline marker values before surgery.

Today, important methods used in the detection of lung cancer are radiology and/or computed tomography (CT) scans. Small nodules, i.e. small regions of suspect tissue can be visualized by these methods. However, many of these nodules - more than 90% with CT - represent benign tissues changes, and only a minority of nodules represents cancerous tissue. Use of the marker APEX may aid in the differentiation of benign versus malign nodules.

In a preferred embodiment the marker APEX is used in an immunohistological method in order to establish or confirm different histological types of LC.

Since APEX as a single marker might be superior to other LC markers like CEA or NSE it has to be expected that APEX will be used as a diagnostic aid, especially by establishing a baseline value before surgery. The present invention thus also relates to the use of APEX for establishing a baseline value before surgery for LC.

### Prognosis:

Prognostic indicators can be defined as clinical, pathological, or biochemical features of cancer patients and their tumors that predict with a certain likelihood the disease outcome. Their main use is to help to rationally plan patient management, i.e. to avoid undertreatment of aggressive disease and overtreatment of indolent disease, respectively. Molina R. et al., Tumor Biol. (2003) 24:209-218 evaluated the prognostic value of CEA, CA 125, CYFRA 21-1, SSC and NSE in NSCLC. In their study abnormal serum levels of the markers NSE, CEA, and LDH (lactate dehydrogenase) appeared to indicate shorter survival.

As APEX alone significantly contributes to the differentiation of LC patients from healthy controls, it has to be expected that it will aid in assessing the prognosis of patients suffering from LC. The level of preoperative APEX will most likely be combined with one or more other marker for LC and/or the TNM staging system. In a preferred embodiment APEX is used in the prognosis of patients with LC.

### Monitoring of Chemotherapy:

Merle, P. et al., Int. J. of Biological Markers (2004) 19:310-315 have evaluated CYFRA 21-1 serum level variations in patients with locally advanced NSCLC treated with induction chemotherapy. They conclude that early monitoring of CYFRA 21-1 serum levels may be a useful prognostic tool for tumor response and survival in stage III NSCLC patients. In addition, reports have described the use of CEA in monitoring the treatment of patients with LC (Fukasawa T. et al., Cancer & Chemotherapy (1986) 13:1862-1867). Most of these studies were retrospective, non-randomized and contained small numbers of patients. As in the case of the studies with CYFRA 21-1 the CEA studies suggested: a) that patients with a decrease in CEA levels while receiving chemotherapy generally had a better outcome than those patients whose CEA levels failed to decrease and (b) for almost all patients, increases in CEA levels were associated with disease progression.

It is expected that APEX will be at least as good a marker for monitoring of chemotherapy as CYFRA 21-1 or CEA, respectively. The present invention therefore also relates to the use of APEX in the monitoring of LC patients under chemotherapy.

### Follow-up:

A large portion of LC patients who undergo surgical resection aimed at complete removal of cancerous tissue later develop recurrent or metastatic disease (Wagner, H., Chest (2000) 117:110-118; Buccheri, G. et al., Ann. Thorac. Surg. (2003) 75:973-980). Most of these relapses occur within the first 2-3 years after surgery. Since recurrent/metastatic disease is invariably fatal if detected too late, considerable research has focused on LC relapse at an early and thus potentially treatable stage.

Consequently, many LC patients undergo a postoperative surveillance program which frequently includes regular monitoring with CEA. Serial monitoring with CEA one year after surgical resection has been shown to detect an early postoperative recurrent/metastatic disease with a sensitivity of approximately 29 %, at a specificity of approximately 97 %, even in the absence of suspicious symptoms or signs (Buccheri, G. et al., Ann. Thorac. Surg. (2003) 75:973-980). Thus, the follow-up of patients with LC after surgery is one of the most important fields of use for an appropriate biochemical marker. Due to the high sensitivity of APEX in the LC patients investigated it is likely that APEX alone or in combination with one or more other marker will be of great help in the follow-up of LC patients, especially in LC patients after surgery. The use of a marker panel comprising APEX and one or more other marker of LC in the follow-up of LC patients represents a further preferred embodiment of the present invention.

The present invention in a preferred embodiment relates to the use of APEX in the diagnostic field of LC or in the assessment of LC, respectively.

In yet a further preferred embodiment the present invention relates to the use of APEX as a marker molecule for lung cancer in combination with one or more marker molecules for lung cancer in the assessment of lung cancer from a liquid sample obtained from an individual. Preferred selected other LC markers with which the measurement of APEX may be combined are CYFRA 21-1, CEA, NSE, proGRP, and/or SCC. Yet further preferred the marker panel used in the assessment of LC comprises APEX and at least one other marker molecule selected from the group consisting of CYFRA 21-1 and CEA.

As the skilled artisan will appreciate there are many ways to use the measurements of two or more markers in order to improve the diagnostic question under investigation. In a quite simple, but nonetheless often effective approach, a positive result is assumed if a sample is positive for at least one of the markers investigated. This may e.g. the case when diagnosing an infectious disease, like AIDS.

Frequently, however, the combination of markers is evaluated. Preferably the values measured for markers of a marker panel, e.g. for APEX and CYFRA 21-1, are mathematically combined and the combined value is correlated to the underlying diagnostic question. Marker values may be combined by any appropriate state of the art mathematical method. Well-known mathematical methods for correlating a marker combination to a disease employ methods like, discriminant analysis (DA) (i.e. linear-, quadratic-, regularized-DA), Kernel Methods (i.e. SVM), Nonparametric Methods (i.e. k-Nearest-Neighbor Classifiers), PLS (Partial Least Squares), Tree-Based Methods (i.e. Logic Regression, CART, Random Forest Methods, Boosting/Bagging Methods), Generalized Linear Models (i.e. Logistic Regression), Principal Components based Methods (i.e. SIMCA), Generalized Additive Models, Fuzzy Logic based Methods, Neural Networks and Genetic Algorithms based Methods. The skilled artisan will have no problem in selecting an appropriate method to evaluate a marker combination of the present invention. Preferably the method used in correlating the marker combination of the invention e.g. to the absence or presence of LC is selected from DA (i.e. Linear-, Quadratic-, Regularized Discriminant Analysis), Kernel Methods (i.e. SVM), Nonparametric Methods (i.e. k-Nearest-Neighbor Classifiers), PLS (Partial Least Squares), Tree-Based Methods (i.e. Logic Regression, CART, Random Forest Methods, Boosting Methods), or Generalized Linear Models (i.e. Logistic Regression). Details relating to these statistical methods are found in the following references: Ruczinski, I., et al, J. of Computational and Graphical Statistics, 12 (2003) 475-511; Friedman, J. H. , J. of the American Statistical Association 84 (1989) 165-175; Hastie, Trevor, Tibshirani, Robert, Friedman, Jerome, The Elements of Statistical Learning, Springer Series in Statistics, 2001; Breiman, L., Friedman, J. H., Olshen, R. A., Stone, C. J. (1984) Classification and regression trees, California: Wadsworth; Breiman, L., Random Forests, Machine Learning, 45 (2001) 5-32; Pepe, M. S., The Statistical Evaluation of Medical Tests for Classification and Prediction, Oxford Statistical Science Series, 28 (2003); and Duda, R. O., Hart, P. E., Stork, D. G., Pattern Classification, Wiley Interscience, 2nd Edition (2001).

It is a preferred embodiment of the invention to use an optimized multivariate cut-off for the underlying combination of biological markers and to discriminate state A from state B, e.g. diseased from healthy. In this type of analysis the markers are no longer independent but form a marker panel.

Accuracy of a diagnostic method is best described by its receiver-operating characteristics (ROC) (see especially Zweig, M. H., and Campbell, G., Clin. Chem. 39 (1993) 561-577). The ROC graph is a plot of all of the sensitivity/specificity pairs resulting from continuously varying the decision threshold over the entire range of data observed.

The clinical performance of a laboratory test depends on its diagnostic accuracy, or the ability to correctly classify subjects into clinically relevant subgroups. Diagnostic accuracy measures the test's ability to correctly distinguish two different conditions of the subjects investigated. Such conditions are for example health and disease or benign versus malignant disease.

In each case, the ROC plot depicts the overlap between the two distributions by plotting the sensitivity versus 1 - specificity for the complete range of decision thresholds. On the y-axis is sensitivity, or the true-positive fraction [defined as (number of true-positive test results)/(number of true-positive + number of false-negative test results)]. This has also been referred to as positivity in the presence of a disease or condition. It is calculated solely from the affected subgroup. On the x-axis is the false-positive fraction, or 1 - specificity [defined as (number of false-positive results)/(number of true-negative + number of false-positive results)]. It is an index of specificity and is calculated entirely from the unaffected subgroup. Because the true- and false-positive fractions are calculated entirely separately, by using the test results from two different subgroups, the ROC plot is independent of the prevalence of disease in the sample. Each point on the ROC plot represents a sensitivity/1-specificity pair corresponding to a particular decision threshold. A test with perfect discrimination (no overlap in the two distributions of results) has an ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. (If the ROC plot falls completely below the 45° diagonal, this is easily remedied by reversing the criterion for "positivity" from "greater than" to "less than" or vice versa.) Qualitatively, the closer the plot is to the upper left corner, the higher the overall accuracy of the test.

One preferred way to quantify the diagnostic accuracy of a laboratory test is to express its performance by a single number. Such an overall parameter e.g. is the so-called "total error" or alternatively the "area under the curve = AUC". The most common global measure is the area under the ROC plot. By convention, this area is always ≥ 0.5 (if it is not, one can reverse the decision rule to make it so). Values range between 1.0 (perfect separation of the test values of the two groups) and 0.5 (no apparent distributional difference between the two groups of test values). The area does not depend only on a particular portion of the plot such as the point closest to the diagonal or the sensitivity at 90% specificity, but on the entire plot. This is a quantitative, descriptive expression of how close the ROC plot is to the perfect one (area = 1.0).

In a preferred embodiment the present invention relates to a method for improving the diagnostic accuracy for LC versus healthy controls by measuring in a sample the concentration of at least APEX and CYFRA 21-1, and optionally of CEA, proGRP, NSE, and/or SCC, respectively and correlating the concentrations determined to the presence or absence of LC, the improvement resulting in more patients being correctly classified as suffering from LC versus healthy controls as compared to a classification based on any single marker investigated alone.

In a preferred method according to the present invention at least the concentration of the biomarkers APEX and CYFRA 21-1, respectively, is determined and the marker combination is used in the assessment of LC.

In a further preferred method according to the present invention at least the concentration of the biomarkers APEX and CEA, respectively, is determined and the marker combination is used in the assessment of LC.

In a further preferred method according to the present invention at least the concentration of the biomarkers APEX, CYFRA 21-1, and CEA, respectively, is determined and the marker combination is used in the assessment of LC. In a further preferred method according to the present invention at least the concentration of the biomarkers APEX, CYFRA 21-1, and proGRP, respectively, is determined and the marker combination is used in the assessment of LC.

In yet a further preferred method according to the present invention at least the concentration of the biomarkers APEX, CYFRA 21-1, and SCC, respectively, is determined and the marker combination is used in the assessment of LC.

The following examples and the figure are provided to aid the understanding of the present invention, the true scope of which is set forth in the appended claims.

### Description of the Figures

**Figure 1** Figure 1 shows a plot of the receiver operator characteristics (ROC) for the assessment of 60 samples obtained from patients with LC as compared to 60 control samples obtained from 30 obviously healthy individuals and 30 apparently healthy smokers.
**Figure 2** Figure 2 shows a Western Blot analysis of lung cancer tissue lysates. 5 µg total protein of 20 lung cancer tissue lysates (10 adeno and 10 squamous cell CA) and matched control tissue lysates were analyzed as described in Example 5.
M = molecular weight marker;
T = tumour tissue lysate;
N = matched control tissue lysate;
PP = plasma pool derived from healthy donors (the band in the range of about 60 kD presumably is due to a non-specific back-ground reaction)
rec. AG = recombinantly produced APEX (10, 3 or 1 ng per lane);
arrows indicate the position of APEX.

### Example 1

### Identification of APEX as a marker for lung cancer

### Sources of tissue:

In order to identify tumor-specific proteins as diagnostic markers for lung cancer, analysis of two different kinds of tissue using proteomics methods is performed.

In total, tissue specimen from 11 patients suffering from lung cancer are analyzed. From each patient two different tissue types are collected from therapeutic resections: tumor tissue (>80% tumor) (T) and adjacent healthy tissue (N) The latter one serves as matched healthy control samples. Tissues are immediately snap frozen after resection and stored at -80°C before processing. Tumors are diagnosed by histopathological criteria.

### Tissue preparation:

0.8-1.2 g of frozen tissue are cut into small pieces, transferred to the chilled grinding jar of a mixer ball mill and completely frozen by liquid nitrogen. The tissue is pulverized in the ball mill, dissolved in the 10-fold volume (w/v) of lysis buffer (40 mM Na-citrate, 5 mM MgCl₂, 1% Genapol X-080, 0.02% Na-azide, Complete^{®} EDTA-free [Roche Diagnostics GmbH, Mannheim, Germany, Cat. No. 1 873 580]) and subsequently homogenized in a Wheaton^{®} glass homogenizer (20 x loose fitting, 20 x tight fitting). The homogenate is subjected to centrifugation (10' at 5,000 x g), the supernatant is transferred to another vial and again subjected to centrifugation (15' at 20,000 x g). The resulting supernatant contains the soluble proteins and is used for further analysis.

### Sample preparation for LC-ESI-MSMS-analysis:

The protein concentration of the soluble protein fraction is determined using Bio-Rad protein assay^{®} (Cat.No. 500-0006; Bio-Rad Laboratories GmbH, München, Germany) following the instructions of the supplier's manual. To a volume corresponding to 200 µg of protein 4 ml reduction buffer (9 M urea, 2 mM DTT, 100 mM KH₂PO₄, NaOH pH 8.2) is added and incubated for 1 hour. This solution is concentrated to 50 µl in an Amicon^{®} Ultra device (Millipore GmbH, Schwalbach, Germany), and for alkylation transferred into 0.5 ml sample buffer (9 M urea, 4 mM iodoacetamide, 100 mM KH₂PO₄, NaOH pH 8.2) and incubated for 6 hours. After alkylation the solution is concentrated in an Amicon^{®} Ultra device to 50 µl and 0.5 ml 9 M urea 10 mM KH₂PO₄, NaOH pH 8.2, are added and the solution is again concentrated to 50 µl. This procedure is repeated twice. Subsequently the final 50 µl are diluted to 990 µl with 4 µg trypsin (Proteomics grade, Roche Diagnostics GmbH, Mannheim, Germany) in water and digested over night.

### LC-ESI-MSMS-analysis:

The tryptic digest (100µl) is separated by two-dimensional HPLC (MudPIT) on a Nano-LC system (Ultimate, Famos, Switchos; LC Packings, Idstein, Germany). The separation is performed with self packed two-dimensional columns (Fused silica: PicoFrit 75µm, New Objective; RP: ProntoSil 120-5-C18 AQ+, Bischoff; SCX: Partisil 10, Whatman). 11 SCX fractions are generated by step elution with successively increasing amounts of NH₄Ac (0 to 1500 mM). They are further separated on the RP part of the column and online analyzed using data dependent scans with an ESI-MS ion trap (LCQ deca XP; Thermo Electron, Massachusetts, USA; see Table 1 for parameters). For each sample three runs are performed. The raw data are processed with a non-commercial Roche own data managing system using Sequest as base algorithm (Parameters see Tab. 1). The resulting lists of identified peptides and proteins from replicate runs where combined.

The protein APEX is identified by aid of the sequences identified and given in Tab. 2.

### Detection of APEX as a marker for lung cancer:

For each patient the identified proteins and the number of corresponding peptides from the tumor sample are compared to the accordant results from adjacent normal tissue. By this means, protein APEX is found to be specifically present or to be strongly abundant in tumor tissue and not to be detectable or to be barely detectable in healthy control tissue.

**Table 1: MSMS-data acquisition and database search parameters**

| **MSMS-data acquisition** | **MS exclusion** | **350-2000 Da for precursor ions** |
|---|---|---|
| | Repeat count | 2 |
| | Repeat duration | 0.25 min |
| | Exclusion list size | 50 |
| | Exclusion duration | 5 min |
| | Exclusion mass width | low 0.5 Da, high 1.5 Da |
| Sequest | Number of ions | 30 |
| | Minimal ion intensity | 10.000 counts |
| | Precursor mass tolerance | 1.5 Da |
| | Fragment mass tolerance | 1.0 Da |
| | X_{corr} | >1.8; 2.3, 2.8 (z=1;2;3) |
| | dCn | > 0.1 |
| | Sp | > 500 |
| Databases | | Humangp (assembled by Roche Bioinformatics) |

The protein APEX is strongly over-represented in tumor tissue from patients suffering from lung cancer. The following peptide sequences of the protein APEX are identified by database search form LCQ-MS²-data in tumor tissue: The following sequences derived from APEX are identified using the above described method.

**Table 2: Sequences identified by ESI-MSMS**

| **Sequence** | **Stretch of amino acid from APEX** |
|---|---|
| GAVAEDGDELRTEPEAK | 7-23 |
| GLDWVKEEAPDILCLQETK | 79-97 |
| KPLVLCGDLNVAHEEIDLR | 202-220 |
| QGFGELLQAVPLADSFR | 237-253 |
| VSYGIGDEEHDQEGR | 241-255 |
| LDYFLLSHSLLPALCDSK | 281-298 |
| ALGSDHCPITLYLAL | 303-317/ C-Terminus |

APEX could be identified in tumor tissue lysate samples from 5 of 8 patients with lung adenocarcinoma. In normal tissue lysates APEX could not be identified.

### Example 2

### Generation of antibodies to the lung cancer marker protein APEX

Polyclonal antibody to the lung cancer marker protein APEX is generated for further use of the antibody in the measurement of serum and plasma and blood levels of APEX by immunodetection assays, e.g. Western Blotting and ELISA.

### Recombinant protein expression in E. coli:

In order to generate antibodies against APEX, the recombinant antigen is produced in *E.coli*: Therefore, the APEX coding region is PCR amplified from the full-length cDNA clone IRAT p970H075D obtained from the German Resource Center for Genome Research (RZPD, Berlin, Germany) using the primers:
Forward primer LC38for-EcoRI:
   5' ACGTACGTGA ATTCATTAAA GAGGAGAAAT TAACTATGAG AGGATCGCAT CACCATCACC ATCACATTGA AGGCCGTCCG AAGCGTGGGA AAAAGG (SEQ ID NO: 2 / *Eco*RI - site underlined and start codon underlined),
Reverse primer LC38rev-*Bam*HI:
   5' CGTACGTGGA TCCTCATTAC AGTGCTAGGT ATAGGGTGAT AGG (SEQ ID NO: 3 / *Bam*HI - site underlined).

The forward primer features (besides the EcoRI cloning and ribosomal binding sites) oligonucleotides coding for an N-terminal MRGSHHHHHHIEGR peptide extension (SEQ ID NO: 4) introduced in-frame to the APEX polypeptide. The *Eco*RI/*Bam*HI digested PCR fragment is ligated into the corresponding pQE-30 (Qiagen, Hilden, Germany) vector fragment which is subsequently transformed into *E.coli* XL1-blue competent cells. After sequence analysis, the plasmid is transformed into *E.coli* BL21 competent cells for expression under the IPTG -inducible T5 promoter of the pQE vector series following the manufacturer's instructions.

For purification of the MRGSHHHHHHIEGR-APEX fusion protein, 1 I of an over-night induced bacterial culture is pelleted by centrifugation and the cell pellet is resuspended in 20 mM sodium-phosphate buffer, 500 mM sodium chloride, pH 7.4 containing 1mg/ml lysozyme and Complete™ EDTA-free protease inhibitor tablets. The cells are disrupted by ultrasonication and insoluble material is pelleted by centrifugation and the supernatant is applied to Ni-nitrilotriacetic acid (Ni-NTA) metal-affinity chromatography: The column is washed with several bed volumes of lysis buffer followed by washes with 20 mM sodium-phosphate buffer, 500 mM sodium chloride, 20 mM imidazol, pH 7.4. Finally, bound antigen is eluted with an imidazol gradient from 20 to 500 mM in 20 mM sodium-phosphate buffer, 500 mM sodium chloride, pH 7.4 and stored in 75 mM HEPES-buffer, pH 7.5, 100 mM sodium chloride, 1 mM EDTA, 6.5 % sucrose at 4°C.

### Generation of polyclonal antibodies:

### a) Immunization

For immunization, a fresh emulsion of the protein solution (100 µg/ml protein APEX) and complete Freund's adjuvant at the ratio of 1:1 is prepared. Each rabbit is immunized with 1 ml of the emulsion at days 1, 7, 14 and 30, 60 and 90. Blood is drawn and resulting anti-APEX serum is used as described hereinbelow.

### b) Purification of IgG (immunoglobulin G) from rabbit serum by sequential precipitation with caprylic acid and ammonium sulfate

One volume of rabbit serum is diluted with 4 volumes of acetate buffer (60 mM, pH 4.0). The pH is adjusted to 4.5 with 2 M Tris-base. Caprylic acid (25 µl/ml of diluted sample) is added drop-wise under vigorous stirring. After 30 min the sample is centrifuged (13 000 x g, 30 min, 4°C), the pellet discarded and the supernatant collected. The pH of the supernatant is adjusted to 7.5 by the addition of 2 M Tris-base.

The immunoglobulin in the supernatant is precipitated under vigorous stirring by the drop-wise addition of a 4 M ammonium sulfate solution to a final concentration of 2 M. The precipitated immunoglobulins are collected by centrifugation (8000 x g, 15 min, 4°C).

The supernatant is discarded. The pellet is dissolved in 10 mM NaH₂PO₄/NaOH, pH 7.5, 30 mM NaCl and exhaustively dialyzed. The dialysate is centrifuged (13 000 x g, 15 min, 4°C) and filtered (0.2 µm).

### c) Biotinylation of polyclonal rabbit IgG:

Polyclonal rabbit IgG is brought to 10 mg/ml in 10 mM NaH₂PO₄/NaOH, pH 7.5, 30 mM NaCl. Per ml IgG solution 50 µl Biotin-N-hydroxysuccinimide (3.6 mg/ml in DMSO) are added. After 30 min at room temperature, the sample is chromatographed on Superdex 200 (10 mM NaH₂PO₄/NaOH, pH 7.5, 30 mM NaCl). The fractions containing biotinylated IgG are collected.

### d) Immunosorption of polyclonal rabbit IgG:

For the APEX immunosorber 10 mg purified recombinant APEX is coupled to 1ml CNBr-activated Sepharose™ 4B (GE Healthcare, Germany Catalog No. 17-04-30-01) according to the manufacturer's protocol. This affinity column is loaded with 100 mg polyclonal rabbit IgG in PBS, 0.05 % Tween 20 followed by washes with a) PBS, b) 0.5 M sodium chloride, 0.05% Tween 20, c) 30 mM sodium chloride. The bound fraction is eluted with 0.5 M glycine, 150 mM sodium chloride adjusted to pH 2.1 with hydrochloric acid and immediately brought to a neutral pH by the addition of 1 M Tris-base. The eluate is concentrated to 10 mg/ml and chromatographed on a TSK-Gel® G3000SW gelfiltration column (Sigma-Aldrich, Germany, catalogue No. 815103) in PBS. The fractions containing IgG monomers are collected.

### Example 3

### ELISA for the measurement of APEX in human serum and plasma samples.

For detection of APEX in human serum or plasma, a sandwich ELISA is developed. For capture of the antigen, anti-APEX polyclonal antibody (see Example 2) is immunosorbed and for detection of the antigen anti-APEX polyclonal antibody is conjugated with biotin.

96-well microtiter plates are incubated with 100 µl immunosorbed anti-APEX polyclonal antibody for 60 min at 5 µg/ml in 150 mM disodium carbonate, 350mM sodium hydrogen carbonate. Subsequently plates are washed three times with PBS, 0.05% Tween 20. Wells are then incubated for 2 h with either a serial dilution of the recombinant protein (see Example 2) as standard antigen or with diluted plasma samples from patients together with 5 µg/ml biotinylated anti-APEX polyclonal antibody. Incubation was in 10 mM phosphate, pH 7.4, 1% BSA, 0.9% NaCl and 0.1% Tween 20. Thereafter, plates are washed three times to remove unbound components. In a next step, wells are incubated with 20 mU/ml anti-biotin-POD conjugate for 60 min in 10 mM phosphate, pH 7.4, 1% BSA, 0.9% NaCl and 0.1% Tween 20. Plates are subsequently washed three times with the same buffer. For detection of bound antigen-antibody complexes, wells are incubated with 100 µl ABTS solution (Roche Diagnostics GmbH, Mannheim, Germany, Catalog No. 11685767) and OD is measured after 30-60 min at 405 nm with an ELISA reader.

### Example 4

### Study population

Samples derived from 60 well-characterized NSCLC patients (30 adeno-CA, 30 squamous cell CA) with the UICC classification given in Table 3 are used.

**Table 3: Study population**

| **Stage according to UICC** | **Number of samples** |
|---|---|
| UICC I/II | 24 |
| UICC III | 17 |
| UICC IV | 19 |
| obviously healthy blood donors | 30 |
| apparently healthy smokers | 30 |

The level of APEX in the LC samples of Table 3 is evaluated in comparison to 60 control samples obtained from 30 obviously healthy individuals and 30 apparently healthy smokers without any known malignant lung disease (= control cohort).

The level of APEX in the LC samples of Table 3 is increased as compared to the level of APEX in control samples.

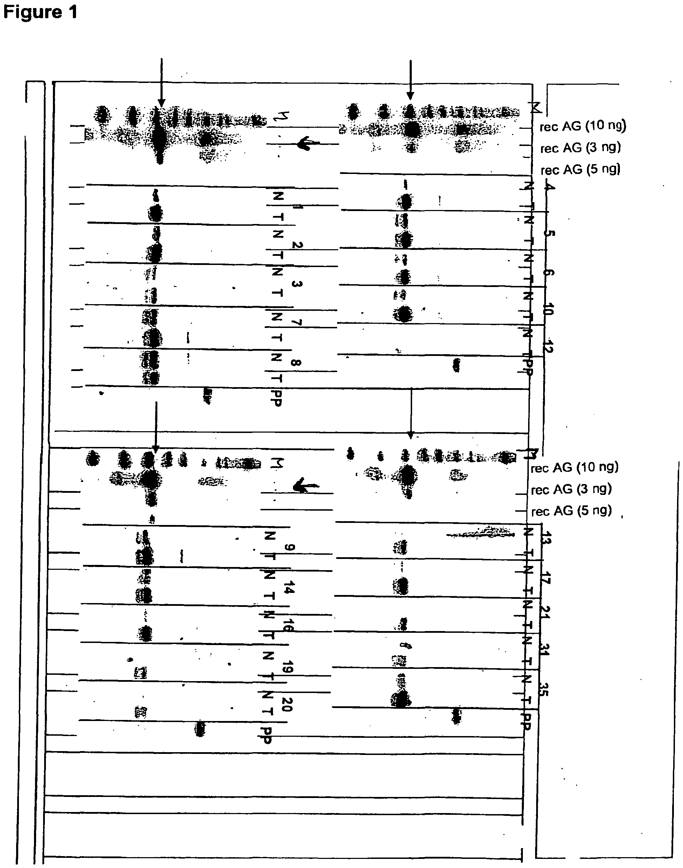

ROC-analysis is performed according to Zweig, M. H., and Campbell, supra. Discriminatory power for differentiating patients in the LC group from healthy individuals as measured by the area under the curve is found to be 92 % for LC vs. healthy controls (Figure 1).

At 95% specificity the sensitivity of all LC samples was 70%, for adenocarcinomas 67% and for squamous cell carcinoma 73% respectively.

### Example 5

### Western Blotting for the detection of APEX in human lung cancer tissue using polyclonal antibody as generated in Example 2.

Tissue lysates from tumor samples and healthy control samples are prepared as described in Example 1, "Tissue preparation".

SDS-PAGE and Western-Blotting are carried out using reagents and equipment of Invitrogen, Karlsruhe, Germany. For each tissue sample tested, 15 µg of tissue lysate are diluted in reducing NuPAGE^{®} (Invitrogen) SDS sample buffer and heated for 10 min at 95°C. Samples are run on 4-12% NuPAGE^{®} gels (Tris-Glycine) in the MES running buffer system. The gel-separated protein mixture is blotted onto nitrocellulose membranes using the Invitrogen XCell II^{®} Blot Module (Invitrogen) and the NuPAGE^{®} transfer buffer system. The membranes are washed 3 times in PBS/0.05% Tween-20 and blocked with Roti^{®}-Block blocking buffer (A151.1; Carl Roth GmbH, Karlsruhe, Germany) for 2 h. The primary antibody, polyclonal rabbit anti-APEX serum (generation described in Example 2), is diluted 1:10,000 in Roti®-Block blocking buffer and incubated with the membrane for 1 h. The membranes are washed 6 times in PBS/0.05% Tween-20. The specifically bound primary rabbit antibody is labeled with an POD-conjugated polyclonal sheep anti-rabbit IgG antibody, diluted to 10 mU/ml in 0.5 x Roti®-Block blocking buffer. After incubation for 1 h, the membranes are washed 6 times in PBS/0.05% Tween-20. For detection of the bound POD-conjugated anti-rabbit antibody, the membrane is incubated with the Lumi-Light^{PLUS} Western Blotting Substrate (Order-No. 2015196, Roche Diagnostics GmbH, Mannheim, Germany) and exposed to an autoradiographic film.

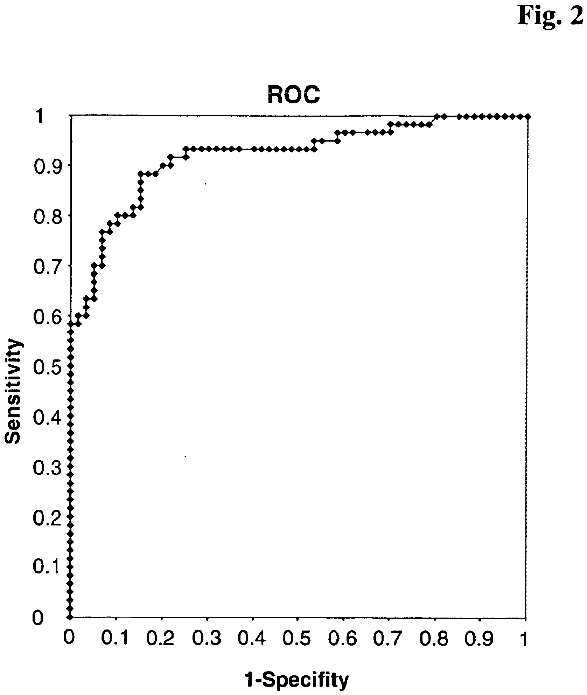

Signal intensity for APEX is increased in 16 out of 20 tumor tissue lysates as obtained from 20 different LC patients (Fig. 2). The expression level was >600 ng/mg. Thus, the increased abundance of APEX in lung cancer tissue as detected by MALDI in Example 1 is confirmed by Western Blotting analyses.

## Claims

1. A method for assessing lung cancer in vitro comprising measuring in a sample the presence and/or concentration of
(a) AP endonuclease (APEX) in a sample selected from tissue extracts and body fluids,
(b) optionally one or more other marker of lung cancer, and
(c) using the measurement result of step (a) and optionally of step (b) in the assessment of lung cancer, wherein detection of increased presence and/or concentration of APEX is indicative for lung cancer.

2. The method according to claim 1, wherein said one or more other marker is selected from the group consisting of the soluble 30 kDa fragment of cytokeratin 19 (CYFRA 21-1), carcinoembryonic antigen (CEA), neuron-specific enolase (NSE), proGRP and squamous cell carcinoma antigen (SCC).

3. The method according to claim 2, wherein said one or more other marker is CYFRA 21-1.

4. The method according to claim 2, wherein said one or more other marker is CEA.

5. The method according to claim 2, wherein said one or more other marker is SCC.

6. Use of APEX in the in vitro assessment of lung cancer in a sample selected from tissue extracts and body fluids, wherein detection of increased presence and/or concentration of APEX is indicative for lung cancer.

7. Use of a marker panel comprising APEX and one or more other marker for lung cancer in the in vitro assessment of lung cancer, in a sample selected from tissue extracts and body fluids, wherein detection of increased presence and/or concentration of APEX is indicative for lung cancer.

8. Use of the marker panel according to claim 7, wherein the one or more other marker is selected from the group consisting of CYFRA 21-1, CEA, NSE, and SCC.

9. Use of a marker panel according to claim 8 comprising at least APEX and CYFRA 21-1.

## Patentansprüche

1. Verfahren zur Einschätzung von Lungenkrebs in vitro, umfassend das Messen in einer Probe des Vorhandenseins und/oder der Konzentration von
(a) AP endonuclease (APEX) in einer Probe ausgewählt aus Gewebeextrakten und Körperflüssigkeiten,
(b) gegebenenfalls einer oder mehrere anderer Marker von Lungenkrebs, und
(c) Verwendung des Messergebnisses aus Schritt (a) und gegebenenfalls aus Schritt (b) zur Einschätzung von Lungenkrebs, wobei die Detektion von erhöhtem Vorhandensein und/oder erhöhter Konzentration von APEX indikativ für Lungenkrebs ist.

2. Verfahren nach Anspruch 1, wobei der eine oder die mehreren anderen Marker ausgewählt sind aus der Gruppe bestehend aus dem löslichen 30 kDa Fragment von Cytokeratin 19 (CYFRA 21-1), carcinoembryonalem Antigen (CEA), neuronspezifischer Enolase (NSE), proGRP und Plattenepithelkarzinom-Antigen (SCC).

3. Verfahren nach Anspruch 2, wobei der eine oder die mehreren anderen Marker CYFRA 21-1 ist.

4. Verfahren nach Anspruch 2, wobei der eine oder die mehreren anderen Marker CEA ist.

5. Verfahren nach Anspruch 2, wobei der eine oder die mehreren anderen Marker SCC ist.

6. Verwendung von APEX in der in vitro Einschätzung von Lungenkrebs in einer Probe ausgewählt aus Gewebeextrakten und Körperflüssigkeiten, wobei die Detektion von erhöhtem Vorhandensein und/oder erhöhter Konzentration von APEX indikativ für Lungenkrebs ist.

7. Verwendung eines Marker-Panels, umfassend APEX und einen oder mehrere andere Marker für Lungenkrebs in der in vitro Einschätzung von Lungenkrebs in einer Probe ausgewählt aus Gewebeextrakten und Körperflüssigkeiten, wobei die Detektion von erhöhtem Vorhandensein und/oder erhöhter Konzentration von APEX indikativ für Lungenkrebs ist.

8. Verwendung des Marker-Panels nach Anspruch 7, wobei der eine oder die mehreren anderen Marker ausgewählt ist/sind aus der Gruppe bestehend aus CYFRA 21-1, CEA, NSE und SCC.

9. Verwendung eines Marker-Panels nach Anspruch 8, umfassend mindestens APEX und CYFRA 21-1.

## Revendications

1. Procédé d'évaluation du cancer du poumon *in vitro* qui comprend la mesure dans un échantillon de la présence et/ou de la concentration de
(a) l'AP endonucléase (APEX) dans un échantillon choisi parmi des extraits tissulaires et des fluides biologiques,
(b) optionnellement un ou plusieurs autres marqueurs du cancer du poumon, et
(c) l'utilisation du résultat de la mesure de l'étape (a) et optionnellement de l'étape
(b) dans l'évaluation du cancer du poumon, dans lequel la détection d'une présence et/ou d'une concentration accrues de l'APEX indique un cancer du poumon.

2. Procédé selon la revendication 1, dans lequel ledit ou lesdits autre(s) marqueur(s) est(sont) choisi(s) dans le groupe constitué du fragment soluble de 30 kDa de la cytokératine 19 (CYFRA 21-1), de l'antigène carcinoembryonnaire (CEA), de l'énolase neurone spécifique (NSE), de proGRP et de l'antigène du carcinome à cellules squameuses (SCC).

3. Procédé selon la revendication 2, dans lequel ledit ou lesdits autre(s) marqueur(s) est(sont) le CYFRA 21-1.

4. Procédé selon la revendication 2, dans lequel ledit ou lesdits autre(s) marqueur(s) est(sont) le CEA.

5. Procédé selon la revendication 2, dans lequel ledit ou lesdits autre(s) marqueur(s) est(sont) le SCC.

6. Utilisation de l'APEX dans l'évaluation *in vitro* du cancer du poumon dans un échantillon choisi parmi des extraits tissulaires et des fluides biologiques, dans laquelle la détection d'une présence et/ou d'une concentration accrues de l'APEX indique un cancer du poumon.

7. Utilisation d'un panel de marqueurs comprenant l'APEX et un ou plusieurs autres marqueurs du cancer du poumon dans l'évaluation *in vitro* du cancer du poumon, dans un échantillon choisi parmi des extraits tissulaires et des fluides biologiques, dans laquelle la détection d'une présence et/ou d'une concentration accrues de l'APEX indique un cancer du poumon.

8. Utilisation du panel de marqueurs selon la revendication 7, dans laquelle le ou les autres marqueurs sont choisis dans le groupe constitué du CYFRA 21-1, du CEA, de la NSE, et du SCC.

9. Utilisation d'un panel de marqueurs selon la revendication 8, qui comprend au moins l'APEX et le CYFRA 21-1.
